# EUROPEAN PATENT APPLICATION

(11) **EP 3 480 594 A2**
(43) Date of publication of application: **08.05.2019**
(21) Application number: 16906781.6
(22) Date of filing: 30.06.2016
(51) Int. Cl.: G01N 33/543, G01N 33/531

(54) **MODIFIED CARDIOLIPIN-COATED MAGNETIC NANOBEADS AND PREPARATION METHOD THEREFOR**

(71) Applicant: Shenzhen Yhlo Biotech Co., Ltd., Shenzhen, Guangdong 518054 (CN)
(72) Inventor: QIAN, Chungen, Shenzhen Guangdong 518054 (CN); ZOU, Dingbiao, Shenzhen Guangdong 518054 (CN); WANG, Gang, Shenzhen Guangdong 518054 (CN); HUANG, Pi'nong, Shenzhen Guangdong 518054 (CN); XIA, Fuzhen, Shenzhen Guangdong 518054 (CN)
(74) Representative: Haseltine Lake LLP
(86) International application number: PCT/CN2016/088014
(87) International publication number: WO 2018/000381

(57) **Abstract**

A modified cardiolipin-coated magnetic nanobead and a method for preparing the same are disclosed. The modified cardiolipin-coated magnetic nanobead comprises a modified cardiolipin, a biotin derivative and a streptavidin magnetic bead; the modified cardiolipin and the biotin derivative are linked together by a -CO-NH-structure; the streptavidin magnetic bead is a streptavidin-binding magnetic nanobead, and the biotin derivatives are linked to streptavidin.

## Description

### TECHNICAL FIELD

The disclosure relates to the field of in vitro detection, in particular to a modified cardiolipin-coated magnetic nanobead and a method for preparing the same.

### BACKGROUND

Anticardiolipin antibody is an antibody capable of reacting with a variety of antigen substances containing a phospholipid structure, and the antigen is a negatively charged phospholipid component participating in a variety of cell membrane compositions. Clinically, anticardiolipin antibody mainly exists in patients with antiphospholipid syndrome, various autoimmune diseases and syphilis infection.

Antiphospholipid syndrome (APS), including a variety of autoimmune diseases, are more common in young people, with a male-to-female incidence ratio of about 2:8. Patients may have one or more manifestations, involving multiple systems and organs and including: venous and arterial thrombosis, thrombocytopenia, habitual abortion, cardiomyopathy, heart disease, cerebral and renal infarction, and pulmonary hypertension. Malignant APS may be manifested by progressive and extensive thrombosis in a short term, leading to multiple organ failure and even death. Antiphospholipid syndrome may not only be secondary to systemic lupus erythematosus or other autoimmune diseases, but also occur alone (primary antiphospholipid syndrome).

Antiphospholipid antibodies may also be produced in a patient suffering from syphilis, a sexually transmitted disease caused by spirochete bacteria, *Treponema pallidum.* More than 5 million people are reported to be infected with syphilis each year, including 30,000 congenitally infected infants. Syphilis may be latent and hidden in patients for many years, and may lead to various clinical manifestations. Patients show no clinical symptoms in a latent period of syphilis, which lasts for life in about two-thirds of untreated patients. An infected person is not infectious during the latent period; however, a child born to a mother in the latent period may be infected with congenital syphilis.

Antiphospholipid antibody assay is widely used in the diagnosis of phospholipid syndrome and non-treponema test for syphilis. This assay has the advantage of inexpensively, fastly and conveniently performing on a large number of samples. In addition, since the antiphospholipid antibody has a concentration that will gradually decrease with successful treatment of syphilis, and a high level of treponemal antibody, which is a specific antibody to syphilis infection, will last for several years or even for life. Therefore, the antiphospholipid antibody assay is considered to be a better choice for monitoring the treatment of syphilis.

A main conventional method of detecting an antiphospholipid antibody is to coat cardiolipin by physical adsorption onto a specific solid, such as an ELISA plate, and then binding the antiphospholipid antibody in a sample to be detected with cardiolipin attached to the ELISA plate, thereby achieving the capture of the antiphospholipid antibody in the sample. The concentration of the antiphospholipid antibody in the sample can be indirectly read by allowing the color development of the captured antiphospholipid antibody and measuring a concentration for luminescence.

Since cardiolipin immobilized by physical adsorption is easily dissociated and detached from the solid plate under an influence of external conditions, such as a solvent, heating and the like, a test product prepared by this method has poor stability.

### SUMMARY

Based on this, it is necessary to provide a well-stabilized modified cardiolipin-coated magnetic nanobead for detecting an anticardiolipin antibody and a method for preparing the same.

A modified cardiolipin-coated magnetic nanobead comprises: a modified cardiolipin, a biotin derivative and a streptavidin magnetic bead;
the modified cardiolipin is obtained by oxidizing a hydrophobic fatty acid side chain of cardiolipin, and the modified cardiolipin contains 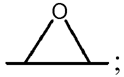 the biotin derivative has a structural formula of 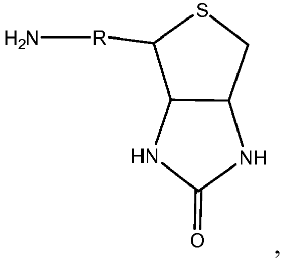 wherein -R- is a saturated alkyl chain having 4 to 20 carbon atoms or a polyethylene glycol chain having 2 to 10 carbon atoms, 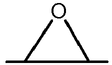 in the modified cardiolipin and -NH₂ in the biotin derivative form a -CO-NH- structure to link the modified cardiolipin and the biotin derivative together;
the streptavidin magnetic bead is a streptavidin-binding magnetic nanobead, and the biotin derivative is linked to streptavidin.

A method for preparing the above modified cardiolipin-coated magnetic nanobead comprises:
reacting cardiolipin with a peroxide sufficiently in the presence of a first solvent to obtain a modified cardiolipin, wherein a hydrophobic fatty acid side chain of modified cardiolipin is oxidized, and the modified cardiolipin contains 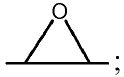
reacting the modified cardiolipin with a biotin derivative sufficiently in the presence of a second solvent to obtain a modified biotin derivative-binding cardiolipin, wherein the biotin derivative has a structural formula of 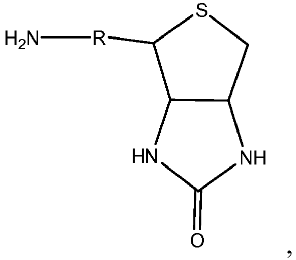 wherein -R- is a saturated alkyl chain having 4 to 20 carbon atoms or a polyethylene glycol chain having 2 to 10 carbon atoms, 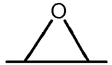 in the modified cardiolipin and -NH₂ in the biotin derivative form a -CO-NH- structure to link the modified cardiolipin and the biotin derivative together; and
mixing and sufficiently reacting the modified biotin derivative-binding cardiolipin with a streptavidin magnetic bead to obtain the modified cardiolipin-coated magnetic nanobead, wherein the streptavidin magnetic bead is a streptavidin-binding magnetic nanobead, and the biotin derivative is linked to streptavidin.

Such a modified cardiolipin-coated magnetic nanobead with direct and strong linkages by chemical bonds between the modified cardiolipin and the biotin derivative and between the biotin derivative and streptavidin on a surface of the streptavidin magnetic bead enables easier control of an amount of modified cardiolipin on the surface of magnetic bead than that in the physical absorption manner, and allows to control the amount of modified cardiolipin and a distance between the magnetic nanobeads by adjusting a length of -R-, so that the amount of modified cardiolipin and a space for binding to an antiphospholipid antibody are better retained. The modified cardiolipin-coated magnetic nanobead can be directly used for the detection of antiphospholipid antibodies, and have higher stability than test products prepared by the conventional method for physically adsorbing cardiolipin.

### DESCRIPTION OF THE DRAWINGS

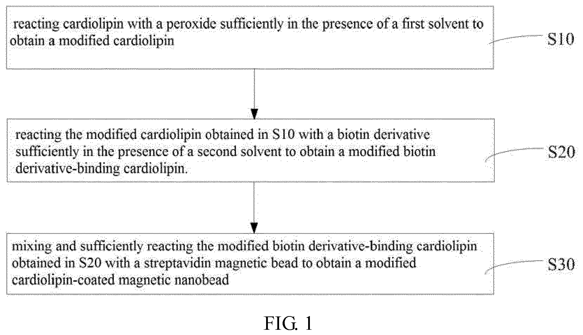

FIG. 1 is a flowchart of a method for preparing a modified cardiolipin-coated magnetic nanobead according to an embodiment.

### DETAILED DESCRIPTION

In order to make the above objects, features and advantages of the present disclosure more clearly understood, specific embodiments of the present disclosure will be described in detail with reference to the accompanying drawings and specific examples. Numerous specific details are set forth in the following description in order to facilitate a thorough understanding of the disclosure. But the disclosure can be practiced in many other ways than those described herein, and those skilled in the art can make similar improvements without departing from the spirit of the disclosure, and therefore the disclosure is not limited by the specific implementation disclosed below.

In an embodiment, a modified cardiolipin-coated magnetic nanobead includes a modified cardiolipin, a biotin derivative and a streptavidin magnetic bead.

Cardiolipin, an ester composed of 3 glycerol, 2 phosphoric acid and 4 unsaturated long chain alkyl groups, has a structure containing 2 hydrophilic cores and 4 hydrophobic side chains. Cardiolipin has a structural formula as follows:

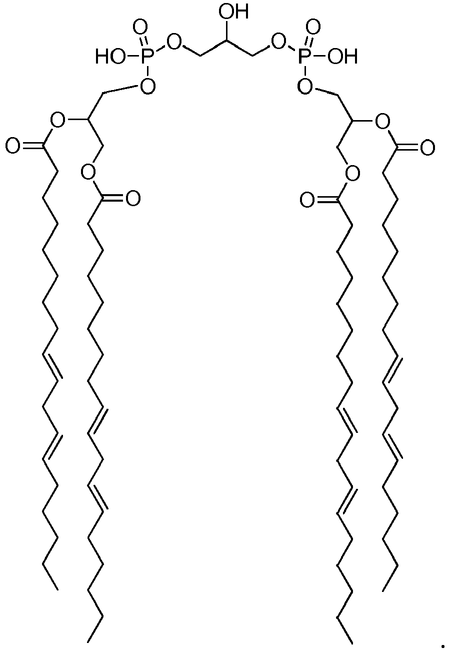

The modified cardiolipin is obtained by oxidizing a hydrophobic fatty acid side chain of cardiolipin, and contains

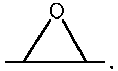

Since cardiolipin has four side chains, which may be simultaneously oxidized when cardiolipin is modified, the modified cardiolipin may contain a plurality of 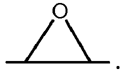 In particular, the modified cardiolipin may comprise 1 to 8

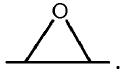

Preferably, the modified cardiolipin contains one

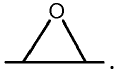

Particularly preferably, the modified cardiolipin has a structural formula as follows:

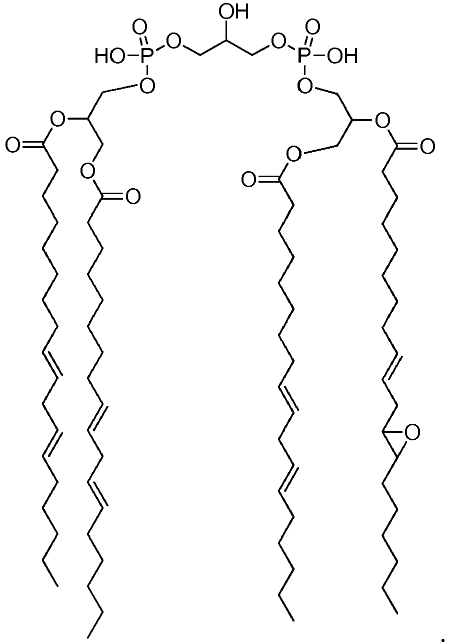

The biotin derivative has a structural formula of 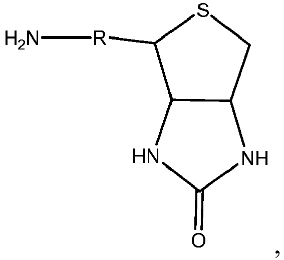 wherein -R- is a saturated alkyl chain having 4 to 20 carbon atoms or a polyethylene glycol chain having 2 to 10 carbon atoms. 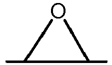 in the modified cardiolipin and -NH₂ in the biotin derivative form a -CO-NH- structure to link the modified cardiolipin and the biotin derivative together.

Preferably, -R- is a saturated alkyl chain containing 4 to 10 carbon atoms.

Particularly preferrably, -R- is -(CH₂)₆-.

Since cardiolipin has a very small molecule size and a seriously insufficient space for modification, the modification of hydrophilic phosphate core will cause a reduced affinity of cardiolipin to antiphospholipid antibody, and even loss of antigenic activity.

The modified cardiolipin at the hydrophilic phosphate core of cardiolipin is retained in the modified cardiolipin-coated magnetic nanobead by modifying the hydrophobic side chain of cardiolipin.

Such a modified cardiolipin-coated magnetic nanobead with direct and strong linkages by chemical bonds between the modified cardiolipin and the biotin derivative and between the biotin derivative and streptavidin on a surface of the streptavidin magnetic bead enables easier control of an amount of modified cardiolipin on the surface of magnetic bead than that in the physical absorption manner, and allows to control the amount of modified cardiolipin and a distance between the magnetic nanobeads by adjusting a length of -R-, so that the amount of modified cardiolipin and a space for binding to an antiphospholipid antibody are better retained. The modified cardiolipin-coated magnetic nanobead can be directly used for the detection of antiphospholipid antibodies, and have higher stability than test products prepared by the conventional method for physically adsorbing cardiolipin.

In addition, since each streptavidin can be tightly bound to 4 biotins, such a modified cardiolipin-coated magnetic nanobead allows a detected signal to be amplified 4 times through biotin-streptavidin amplification effect, greatly improving sensitivity for detecting an antiphospholipid antibody.

As shown in FIG. 1, the method for preparing the above modified cardiolipin-coated magnetic nanobead comprises:
S10, reacting cardiolipin with a peroxide sufficiently in the presence of a first solvent to obtain a modified cardiolipin.

A fatty acid side chain of cardiolipin is oxidized by reaction of cardiolipin with the peroxide.

The peroxide is peroxybenzoic acid, m-chloroperoxybenzoic acid, peroxyacetic acid, or peroxypropionic acid.

A molar ratio of cardiolipin to peroxide ranges from 1:1 to 1:8.

The first solvent is dichloromethane, trichloromethane, chloroform, benzene, or toluene.

In S10, an operation for purifying an intermediate product is further included, and the purifying may be performed by preparative liquid chromatography after extraction with ethyl acetate. After purification, modified cardiolipin having a purity of about 80% may be obtained.

In S10, the reaction is performed at a temperature ranging from 60°C to 100°C.

Since cardiolipin has four side chains, which may be simultaneously oxidized when cardiolipin is modified, the modified cardiolipin may contain a plurality of 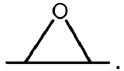 In particular, the modified cardiolipin may comprise 1 to 8

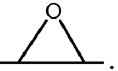

Preferably, the modified cardiolipin contains one

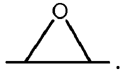

Particularly preferably, the modified cardiolipin has a structural formula as follows:

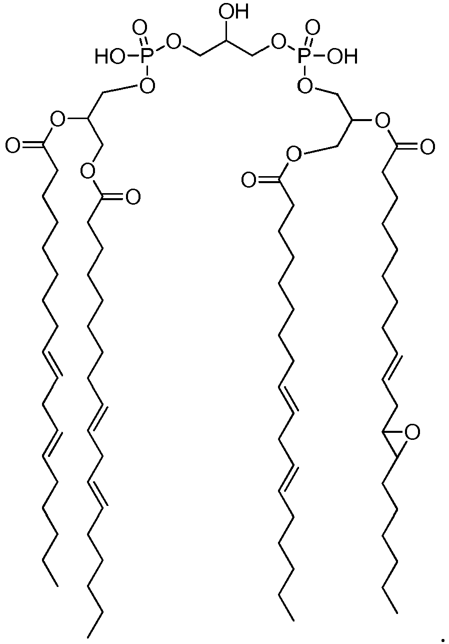

S20, reacting the modified cardiolipin obtained in S10 with a biotin derivative sufficiently in the presence of a second solvent to obtain a modified biotin derivative-binding cardiolipin.

The biotin derivative has a structural formula of 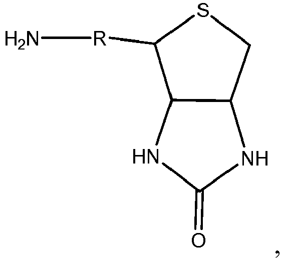 wherein -R- is a saturated alkyl chain having 4 to 20 carbon atoms or a polyethylene glycol chain having 2 to 10 carbon atoms. 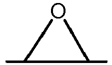 in the modified cardiolipin and -NH₂ in the biotin derivative form a -CO-NH- structure to link the modified cardiolipin and the biotin derivative together.

Preferably, -R- is a saturated alkyl chain containing 4 to 10 carbon atoms.

Particularly preferrably, -R- is -(CH2)₆-.

The biotin derivative can be directly obtained commercially.

In S20, the second solvent is DMSO, DMF, tetrahydrofuran, or phosphate-buffered saline having a pH of 6.5 to 8.5.

In S20, a molar ratio of the modified cardiolipin to the biotin derivative ranges from 1:1.5 to 1:20.

S30, mixing and sufficiently reacting the modified biotin derivative-binding cardiolipin obtained in S20 with a streptavidin magnetic bead to obtain a modified cardiolipin-coated magnetic nanobead.

The streptavidin magnetic bead is a streptavidin-binding magnetic nanobead, and the biotin derivative is linked to streptavidin.

The Streptavidin magnetic bead can be directly obtained commercially, for example, from MagnaBind Corporation (Cat.No. 21344).

In the operation for mixing and sufficiently reacting the modified biotin derivative-binding cardiolipin with the streptavidin magnetic bead, the modified biotin derivative-binding cardiolipin has a concentration of 0.1 mg/mL to 1 mg/mL, and the streptavidin magnetic bead has a concentration of 5 mg/mL to 15 mg/mL.

Such a method for preparing a modified cardiolipin-coated magnetic nanobead with direct and strong linkages by chemical bonds between the modified cardiolipin and the biotin derivative and between the biotin derivative and streptavidin on a surface of the streptavidin magnetic bead enables easier control of an amount of modified cardiolipin on the surface of magnetic bead than that in the physical absorption manner, and allows to control the amount of modified cardiolipin and a distance between the magnetic nanobeads by adjusting a length of -R-, so that the amount of modified cardiolipin and a space for binding to an antiphospholipid antibody are better retained. The modified cardiolipin-coated magnetic nanobead thus prepared can be directly used for the detection of antiphospholipid antibodies, and have higher stability than test products prepared by the conventional method for physically adsorbing cardiolipin.

In addition, since each streptavidin can be tightly bound to 4 biotins, the modified cardiolipin-coated magnetic nanobead prepared allows a detected signal to be amplified 4 times through biotin-streptavidin amplification effect, greatly improving sensitivity for detecting an antiphospholipid antibody.

Specific examples are as follows.

### Example 1: Preparation of modified cardiolipin

0.5 mmol of cardiolipin was dissolved in 1 mL of anhydrous toluene, to which 1.5 mmol of m-chloroperoxybenzoic acid was added, and the temperature was gradually heated to 110°C for 72 hours while stirring. The mixture was reacted at 110°C for 72 hours, and was poured into 25 mL of ice water after cooled to room temperature. The resulting solution was extracted three times with 20 mL of ethyl acetate, and ethyl acetate phases were combined. The combined ethyl acetate phase was washed once with 10 mL of a saturated sodium chloride solution and dried over anhydrous sodium sulfate for 12 hours. Ethyl acetate was evaporated to dryness *in vacuo.* Residual solid was dissolved in a small amount of methanol, and insoluble material was filtered off. The filtrate was purified by preparative liquid chromatography to obtain about 210 mg of modified cardiolipin as colorless oily liquid (MS (ESI⁺, m/z): 1479.83131).

### Example 2: Preparation of modified biotin derivative-binding cardiolipin

To 4 mL of anhydrous DMF solution were sequentially added 200 mg of modified ethoxy cardiolipin and 110 mg of terminally aminated biotin derivative under stirring, and the reaction was performed under stirring for 10 hours at room temperature. The reaction solution was evaporated to dryness *in vacuo.* Residual solid was dissolved in 5 mL of ethyl acetate, insoluble material was filtered off, and the obtained ethyl acetate solvent was evaporated to dryness to obtain a modified biotin derivative-binding cardiolipin as a white solid.

In this example, the biotin derivative has a structural formula of 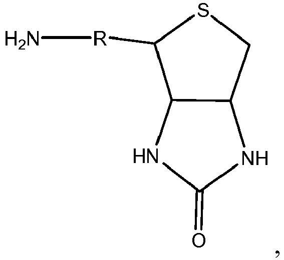 and -R- is -(CH₂)₆.

The modified biotin derivative-binding cardiolipin prepared in this example may be ready for later use without special purification.

### Example 3: preparation of modified cardiolipin-coated streptavidin magnetic nanobead

To 100 µL of phosphate-buffered saline containing 0.2 mg/mL of the modified biotin derivative-binding cardiolipin was added 100 µL of 10 mg/mL streptavidin magnetic bead, and after homogeneous mixing, the mixture was incubated at 37°C for 10 minutes. After magnetic separation, 1 mL of 30 mM Tris buffer was used for redissolution, and the mixture obtained by redissolution was shaken and then magnetically separated again. The obtained solid was redissolved in 5 mL of 30 mM Tris buffer to give a solution of modified cardiolipin-coated streptavidin magnetic nanobead having a concentration of 0.02 mg/mL.

### Example 4: Reaction of modified cardiolipin-coated streptavidin magnetic bead with an antiphospholipid sample

To 200 µL of each solution of modified cardiolipin-coated streptavidin magnetic nanobead prepared in Examples 1 to 3 was added 5 µL of antiphospholipid antibody serum sample and the mixture was incubated at 37°C for 30 minutes. After magnetic separation, the modified cardiolipin-coated streptavidin magnetic nanobead was redissolved in 200 µL, and a horseradish peroxidase-labeled secondary antibody was added, and after incubation at 37°C for 30 minutes, the mixture was sequentially washed, blended with a TMB substrate solution and incubated for 10 minutes. Then, 100 µL of stop solution was added and OD value was read on the microplate reader within 10 minutes to obtain a luminescence signal value of the sample.

Three cardiolipin-positive serum samples and three cardiolipin-negative serum samples were measured respectively for OD values, and the magnetic bead prepared by conventional physical adsorption method is used as a control for comparing the measured OD values, which are shown in the following Table 1.

**Table 1: measured OD values of different samples in Examples 1 to 3 and control group (physical adsorption method)**

| Sample number | Negative sample 1 | Negative sample 2 | Negative sample 3 | Positive sample 1 | Positive sample 2 | Positive sample 3 |
|---|---|---|---|---|---|---|
| Example 1 | 51 | 63 | 101 | 1121 | 5702 | 3528 |
| Example 2 | 73 | 81 | 124 | 1576 | 6993 | 4367 |
| Example 3 | 65 | 71 | 117 | 1477 | 5982 | 4132 |
| control group | 153 | 240 | 381 | 720 | 2155 | 2716 |

As can be seen from table 1, in the detection of antiphospholipid antibody samples, the luminescence signal of the modified cardiolipin-coated streptavidin magnetic nanobead prepared in Examples 1 to 3 is significantly decreased (by 1 to 4 folds) when measuring the negative samples, and tremendously increased (by 3 to 10 folds) when measuring the positive samples, compared to that of the magnetic bead prepared by physical adsorption method (the control group).

Thus, the modified cardiolipin-coated streptavidin magnetic nanobead prepared in Examples 1 to 3 had significantly improved sensitivity for detecting an antiphospholipid sample compared to the magnetic bead made by conventional physical adsorption method.

The examples described above only show one or more embodiments of the present disclosure, the description of which is more specific and detailed, but is not therefore to be understood as limiting the scope of the disclosure. It should be noted that for those of ordinary skill in the art, several variations and modifications may also be made without departing from the inventive concept, which are within the scope of the present disclosure. Therefore, the scope of protection of this disclosure shall be subject to the appended claims.

## Claims

1. A modified cardiolipin-coated magnetic nanobead comprises: a modified cardiolipin, a biotin derivative and a streptavidin magnetic bead;
the modified cardiolipin is obtained by oxidizing a hydrophobic fatty acid side chain of cardiolipin, and the modified cardiolipin contains 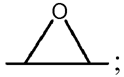 The biotin derivative has a structural formula of 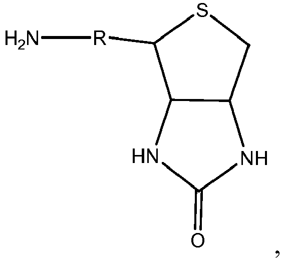 wherein -R- is a saturated alkyl chain having 4 to 20 carbon atoms or a polyethylene glycol chain having 2 to 10 carbon atoms, 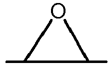 in the modified cardiolipin and -NH₂ in the biotin derivative form a -CO-NH- structure to link the modified cardiolipin and the biotin derivative together;
the streptavidin magnetic bead is a streptavidin-binding magnetic nanobead, and the biotin derivative is linked to streptavidin.

2. The modified cardiolipin-coated magnetic nanobead according to claim 1, wherein the -R- is a saturated alkyl chain containing 4 to 10 carbon atoms.

3. The modified cardiolipin-coated magnetic nanobead according to claim 1, wherein the modified cardiolipin contains one

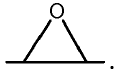

4. The modified cardiolipin-coated magnetic nanobeads according to claim 3, wherein the modified cardiolipin has a structural formula as follows:

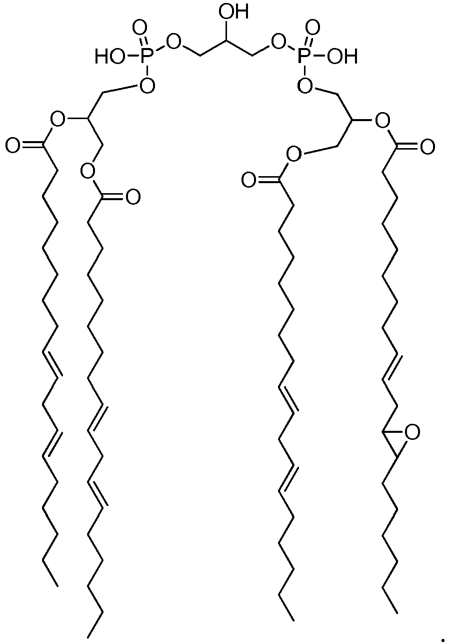

5. A method for preparing the modified cardiolipin-coated magnetic nanobead according to claim 1, comprises:
reacting cardiolipin with a peroxide sufficiently in the presence of a first solvent to obtain the modified cardiolipin, wherein a hydrophobic fatty acid side chain of the modified cardiolipin is oxidized, and the modified cardiolipin contains 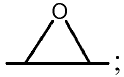
reacting the modified cardiolipin with a biotin derivative sufficiently in the presence of a second solvent to obtain a a modified biotin derivative-binding cardiolipin, wherein the biotin derivative has a structural formula of 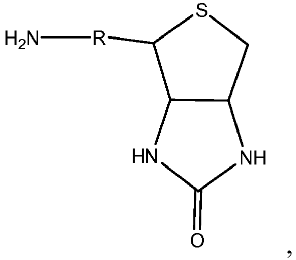 wherein -R- is a saturated alkyl chain having 4 to 20 carbon atoms or a polyethylene glycol chain having 2 to 10 carbon atoms, 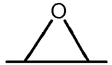 in the modified cardiolipin and -NH₂ in the biotin derivative form a -CO-NH- structure to link the modified cardiolipin and the biotin derivative together; and
mixing and sufficiently reacting the modified biotin derivative-binding cardiolipin with a streptavidin magnetic bead to obtain the modified cardiolipin-coated magnetic nanobead, wherein the streptavidin magnetic bead is a streptavidin-binding magnetic nanobeads, and the biotin derivative is linked to streptavidin.

6. The method for preparing the modified cardiolipin-coated magnetic nanobead according to claim 5, wherein the peroxide is peroxybenzoic acid, m-chloroperoxybenzoic acid, peroxyacetic acid, or peroxypropionic acid, and a molar ratio of cardiolipin to the peroxide ranges from 1:1 to 1:8 in the operation for reacting cardiolipin with the peroxide sufficiently in the presence of the first solvent.

7. The method for preparing the modified cardiolipin-coated magnetic nanobead according to claim 5, wherein the first solvent is dichloromethane, trichloromethane, chloroform, benzene, or toluene in the operation for reacting cardiolipin with the peroxide sufficiently in the presence of the first solvent.

8. The method for preparing the modified cardiolipin-coated magnetic nanobead according to claim 5, wherein the second solvent is DMSO, DMF, tetrahydrofuran, or phosphate-buffered saline having a pH of 6.5 to 8.5 in the operation for reacting the modified cardiolipin with the biotin derivative sufficiently in the presence of the second solvent.

9. The method for preparing the modified cardiolipin-coated magnetic nanobead according to claim 5, wherein a molar ratio of the modified cardiolipin to the biotin derivative ranges from 1:1.5 to 1:20 in the operation for reacting the modified cardiolipin with the biotin derivative sufficiently in the presence of the second solvent.

10. The method for preparing the modified cardiolipin-coated magnetic nanobead according to claim 5, wherein the modified biotin derivative-binding cardiolipin has a concentration of 0.1 mg/mL to 1 mg/mL, and the streptavidin magnetic bead has a concentration of 5 mg/mL to 15 mg/mL in the operation for mixing and sufficiently reacting the modified biotin derivative-binding cardiolipin with the streptavidin magnetic bead.
